(19) Europäisches Patentamt
European Patent Office
Office européen des brevets

(11) Publication number: 0 176 493 B1

(12) EUROPEAN PATENT SPECIFICATION

(45) Date of publication of patent specification: 15.05.91

(51) Int. Cl.[5]: A61K 39/145, A61K 37/02, C07K 15/00, C12P 21/00, C12N 15/44, C12P 19/34, C12N 7/00, C12N 1/20, //C12R1/19

(21) Application number: 85870120.4

(22) Date of filing: 28.08.85

# (54) Vaccinal polypeptides.

(30) Priority: 30.08.84 US 645732

(43) Date of publication of application: 02.04.86 Bulletin 86/14

(45) Publication of the grant of the patent: 15.05.91 Bulletin 91/20

(84) Designated Contracting States: AT BE CH DE FR GB IT LI LU NL SE

(56) References cited:
WO-A-84/00687
DE-A- 3 200 813
US-A- 4 357 421

NATURE, vol. 292, no. 5818, July 2, 1981 (New York, London); G. WINTER et al.:"Nucleotide sequence of the haemagglutinin gene of a human influenza virus H1 subtype"

PROCEEDINGS OF THE NATIONAL ACADEMY OF SCIENCES OF THE UNITED STATES OF AMERICA, vol. 80, no. 19, Octobwer 1983 (Baltimore); J.F. YOUNG et al.:Efficient expression of influenza virus NS1 nonstructuryal proteins in Escherichia coli"

Decision of Technical Board of Appeal 3.3.2 dated 27 January 1988, T 292/85 (Official Journal EPO 7/1989, pp. 275-296

(73) Proprietor: SMITHKLINE BECKMAN CORPORATION
One Franklin Plaza P O Box 7929
Philadelphia Pennsylvania 19103(US)

(72) Inventor: Young, James Francis
10 Holbrook Road
Havertown, Pa, 19083(US)

(74) Representative: Tyrrell, Arthur William Russell et al
SmithKline Beecham, Corporate Patents,
Great Burgh, Yew Tree Bottom Road
Epsom, Surrey KT18 5XQ(GB)

EP 0 176 493 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention relates to vaccine preparation and, more particularly, to preparation of a vaccinal influenza virus polypeptide by recombinant DNA techniques.

### BACKGROUND OF THE INVENTION

Influenza virus infection causes acute respiratory disease in man, swine, horses and fowl, sometimes of pandemic proportions. Influenza viruses are orthomyxoviruses and, as such, have enveloped virions of 80 to 120 nanometers in diameter, with two different glycoprotein spikes. Three types, A, B and C, infect humans. Type A viruses have been responsible for the majority of human epidemics in modern history, although there are also sporadic outbreaks of Type B infections. Known swine, equine and fowl viruses have all been Type A.

The type A viruses are divided into subtypes based the antigenic properties of on the hemagglutinin (HA) and neuraminidase (NA) surface glycoproteins. Within type A, subtypes H1 ("swine flu"), H2 ("asian flu") and H3 ("Hong Kong flu") are predominant in human infections.

Due to genetic drift which, at approximately yearly intervals, affects antigenic determinants in the HA and NA proteins, it has not been possible to prepare a "universal" influenza virus vaccine using conventional killed or attenuated viruses, that is, a vaccine which is non-strain specific. Recently, attempts have been made to prepare such universal, or semi-universal, vaccines from reassortmant viruses prepared by crossing different strains. More recently, such attempts have involved recombinant DNA techniques focusing primarily on the HA protein.

Winter et al., Nature, volume 292, pages 72-75 (1981), report a DNA coding sequence for HA of the A/PR/8/34 strain (H1N1). Percent homology of amino acid and nucleotide sequences of the HA1 and HA2 subunits of this strain were compared to those of representative strains of subtypes H2, H3 and H7.

Baez et al., Nucl. Acids Res., volume 8, pages 5845-5857 (1980), report a DNA coding sequence for the nonstructural (NS) protein of strain A/PR/8/34.

Young et al., in The Origin of Pandemic Influenza Viruses, 1983, edit. by W.G. Laver, Elsevier Science Publishing Co., and Young et al., Proc. Natl. Acad. Sci. USA, volume 80, pages 6105-6109 (1983), report cloning of cDNA from all eight RNA segments from strain A/PR/8/34 in E. coli and report high level expression of the NS1 protein in E. coli.

Emtage et al., U.S. Patent 4,357,421, disclose cloning and expression of a coding sequence for an influenza virus HA gene, and disclose that the HA polypeptide is an antigen which may be administered for vaccine purposes.

The Morbidity and Mortality Weekly Report, volume 33, number 19, pages 253-261, review the most recent prevention and control strategies for influenza virus, including dosage and administration protocol for HA protein-containing human vaccines.

Davis et al., Gene, volume 21, pages 273-284 (1983) report on immune responses in mice to HA-derived polypeptides.

Additional references report cloning and expression of HA, NS and other influenza virus genes of the A/PR/8/34 and other strains. Some of such references are cited hereinbelow.

### SUMMARY OF THE INVENTION

In one aspect, the invention is a vaccine for stimulating protection in animals against infection by influenza virus which comprises a polypeptide, other than an HA protein, having an immunogenic determinant of the HA2 subunit of an HA protein.

In another aspect, the invention is a polypeptide, other than an HA protein, which comprises an immunogenic determinant of the HA2 subunit, which can be used in the vaccine of the invention. The preferred embodiment of this aspect of the invention is herein referred to as the C13 protein.

In other aspects, the invention is a DNA molecule comprising a coding sequence for the vaccinal polypeptide of the invention, including the coding sequence alone or incorporated into a larger molecule, such as a DNA cloning or expression vector, and a microorganism or cell transformed with such DNA molecule.

### DETAILED DESCRIPTION OF THE INVENTION

Oftentimes, immunogenic determinants do not stimulate an immunoprotective response. This is believed to be due, in large part, to a failure to present the determinant to a host's bodily defense system in proper configuration.

As disclosed and fully described hereinbelow, the immunogenic determinant (which may comprise one or more contiguous or separated haptens) of the HA2 subunit of the HA protein, surprisingly, induces a cytotoxic T cell response against different strains within the subtype of origin. Thus, the HA2 immunogenic determinant can provoke a protective immune response, if it is presented in an immunogenic configuration, which is subtype specific, rather than strain specific. For example, the HA2 determinant can be presented in a vaccinal polypeptide comprising the HA2 subunit, that is, substantially the entire HA2 subunit of the HA protein, fused to a second polypeptide which permits an immune response to the immunogenic determinant by causing the HA2 subunit to assume an immunogenic configuration.

Preferably, the polypeptide which permits such immune response to the HA2 immunogenic determinant comprises an amino acid sequence which is expressed at high levels by a recombinant host, prokaryotic or eukaryotic, fused to the N terminus of substantially the entire HA2 subunit. Especially preferred is a polypeptide derived from an influenza virus protein. The vaccinal polypeptide is not the HA protein, because immunoprotective response to the HA protein appears to be strain-specific.

For expression of such vaccinal polypeptide carrying the HA2 immunogenic determinant in E. coli, the polypeptide which permits an immune response to the HA2 determinant is preferably the N terminus of the NS1 influenza virus protein. A particular and preferred embodiment thereof is a protein herein referred to as C13. The C13 protein has the first 81 amino acids of the NS1 protein fused, through a serine and an arginine of HA1 origin, to the entire HA2 subunit.

The vaccinal polypeptide of the invention can be prepared by chemical synthesis techniques. Preferably, however, it is prepared by known recombinant DNA techniques by cloning and expressing within a host microorganism or cell a DNA fragment carrying a coding sequence for the polypeptide. The preferred host is E. coli because it can be used to produce large amounts of desired proteins safely and cheaply.

Coding sequences for the HA2, NS1 and other viral proteins of influenza virus can be prepared synthetically or can be derived from viral RNA, by known techniques, or from available cDNA-containing plasmids. For example, in addition to the above-cited references, a DNA coding sequence for HA from the A/Japan/305/57 strain was cloned, sequenced and reported by Gething et al., Nature, volume 87, pages 301-306 (1980); a HA coding sequence for strain A/NT/60/68 was cloned as reported by Sleigh et al., and by Both et al., both in Developments in Cell Biology, Elsevier Science Publishing Co., pages 69-79 and 81-89, 1980; a HA coding sequence for strain A/WSN/33 was cloned as reported by Davis et al., Gene, volume 10, pages 205-218 (1980); and by Hiti et al., Virology, volume 111, pages 113-124 (1981). An HA coding sequence for fowl plague virus was cloned as reported by Porter et al., and by Emtage et al., both in Developments in Cell Biology, cited above, at pages 39-49 and 157-168. Also, influenza viruses, including other strains, subtypes and types, are available from clinical specimens and from public depositories, such as the American Type Culture Collection, Rockville, Maryland, U.S.A.

Systems for cloning and expressing the vaccinal polypeptide in various microorganisms and cells, including, for example, E. coli, Bacillus, Streptomyces, Saccharomyces and mammalian and insect cells, are known and available from private and public laboratories and depositories and from commercial vendors.

The vaccine of the invention comprises one or more vaccinal polypeptides of the invention, and a carrier or diluent therefor. For example, such vaccine can comprise substantially the entire HA2 subunit from each of several subtypes, each fused to N terminal amino acids of the NS1 protein, which is highly conserved, in normal saline or other physiological solution. Use of an adjuvant, such as aluminum hydroxide, may prove to be desirable. A preferred vaccine comprises three vaccinal polypeptides, each comprising substantially the entire HA2 subunit from one of the H1, H2 and H3 subtypes fused to about the first 80 amino acids of any NS1 protein, as in the case of the C13 protein. Alternatively, a polyvalent vaccine can be prepared from one or more polypeptides of the invention combined with additional immunogens derived from influenza virus or other pathogens, such as subunit or polypeptide antigens or killed viruses or bacteria, to produce a vaccine which can stimulate protection against influenza virus as well as other invasive organisms or viruses. Techniques for formulating such vaccines are well known. For example, the vaccinal polypeptide, and other immunogens in the case of a combination vaccine, can be lyophilized for subsequent rehydration in saline or other physiological solutions.

Dosage and administration protocol can be optimized in accordance with standard vaccination practices. Typically, the vaccine will be administered intramuscularly, although other routes of administration may be used, such as oral, intraocular and intranasal administration. Based on what is known about other

polypeptide vaccines, it is expected that a useful single dosage for average adult humans is in the range of 1.5 to 150 micrograms, preferably 10 to 100 micrograms. The vaccine can be administered initially in late summer or early fall and can be readministered two to six weeks later, if desirable, or periodically as immunity wanes, for example, every two to five years.

The following examples are illustrative, and not limiting, of the invention.

Example 1. Plasmid pM30

Plasmid pAPR701 is a pBR322-derived cloning vector which carries coding regions for the M1 and M2 influenza virus proteins (A/PR/8/34). It is described by Young et al., in The Origin of Pandemic Influenza Viruses, 1983, edited by W.G. Laver, Elsevier Science Publishing Co.

Plasmid pAPR801 is a pBR322-derived cloning vector which carries the NS1 coding region (A/PR/8/34) It is described by Young et al., cited above.

Plasmid pAS1 is a pBR322-derived expression vector which contains the PL promoter, an N utilization site (to relieve transcriptional polarity effects in a presence of N protein) and the cII ribosome binding site including the cII translation initiation codon followed immediately by a Bam HI site. It is described by Rosenberg et al., Methods Enzymol., volume 101, pages 123-138 (1983).

Plasmid pAS1deltaEH was prepared by deleting a non-essential Eco RI-Hin dIII region of pBR322 origin from pAS1. A 1236 base pair Bam HI fragment of pAPR801, containing the NS1 coding region in 861 base pairs of viral origin and 375 base pairs of pBR322 origin, was inserted into the Bam HI site of pAS1deltaEH. The resulting plasmid, pAS1deltaEH/801 expresses authentic NS1 (230 amino acids). This plasmid has an Nco I site between the codons for amino acids 81 and 82 and an Nru I site 3' to the NS sequences. The Bam HI site between amino acids 1 and 2 is retained.

A 571 base pair fragment carrying a coding sequence for the C terminal 50 amino acids of the M1 protein was obtained by restricting pAPR701 with Nco I and Eco R5. This fragment was inserted between the Nco I and Nru I sites in pAS1deltaEH/801 subsequent to deletion of that fragment from the plasmid. The resulting plasmid, pM 30, codes for a fusion protein which is the first 81 amino acids of NS1 fused to the last 50 amino acids of M1. The NcoI and BamHI sites are retained.

Example 2. Plasmid pC13

Plasmid pJZ102 is a pBR322-derived cloning vector which carries a coding region for the entire HA protein (A/PR/8/34). It is described by Young et al., cited in Example 1.

Plasmid pBgl II is a pBR322-derived cloning vector which carries a Bgl II linker at the Nru I site in pBR322.

pJZ102 was cut with MnI I. Bgl II linkers were ligated to all ends and the HA2-containing fragment was reinserted. The 5' junction in the resulting plasmid, pBgl II/HA2, was sequenced as follows:

```
        ___1___ ___2__ ___3___
5'      AGATCTG TCCAGA GGT_ _ _     3'
```

Region 1 is derived from the Bgl II linker and codes for asparagine and leucine. Region 2 is derived from HA1 and codes for serine and arginine. Region 3 is derived from HA2 and codes for all amino acids of the HA2 subunit.

The 3' junction was sequenced as follows:

```
           _____3____  _4_ ______5_______ ___6___
5'  _ _ _ATATGCATC TGA GATTAGAATTTCA CAGATCT
```

Region 4 is the HA2 stop codon. Region 5 is 3' non-coding sequences of viral origin. Region 6 is derived from the Bgl II linker.

A 691 base pair fragment carrying the HA2 coding sequence was obtained by restricting pBgl II/HA2 with Bgl II. The fragment was end-filled with DNApolI (Klenow) and ligated into pAS1deltaEH/80l which had been cut with Nco I and similarly end-filled (Klenow). The resulting plasmid is pC13. The NS1-HA2, blunt ended junction was sequenced as follows:

| | 7 | 1 | 2 | 3 | |
|---|---|---|---|---|---|
| 5' | AAAATGACCATG | GATCTG | TCCAGA | GGT | 3' |

Region 7 is derived from the NS1 gene. Regions 1,2 and 3 are as defined above.

Example 3. Production of the C13 Protein

E. coli host strain N5151, a temperature sensitive lambda lysogen (cI857) was transformed with pC13. Transformants were grown at 32° C to mid-log phase ($A_{260}$ = 0.6) in LB broth supplemented with 100 micrograms/ml of ampicillin. The cultures were then shifted to 42° C to inactivate cI and thus induce synthesis of the C13 protein. After 2 hours at 42° C, the bacteria were collected by centrifugation (3500 rpm, 20 min) and the bacterial pellet was frozen at -20° C.

The pellet was thawed and resuspended in buffer A (5O mM Tris-HCl, pH 8.0, 2Mm EDTA, 1 mM dithiothreitol, 5% (vol/vol) glycerol. Lysozyme was added to a final concentration of 0.2 mg/ml, and the mixture was incubated on ice for 20 minutes. The mixture was then treated in a Waring blender at high speed for six bursts of 15 seconds each. The suspension was then sonicated for one minute with a Branson probe sonifier. The mixture was then centrifuged (15,000 rpm, 30 minutes).

The pellet was resuspended in buffer A by sonication (4 x 15 second bursts). The mixture was then made 0.1% deoxycholate and stirred for one hour at 4° C. The mixture was centrifuged (15,000 rpm, 30 minutes) and the protein was pelleted. The deoxycholate treatment was repeated and the resulting pellet was then resuspended in buffer A by sonication. The suspension was then made 1% Triton X-100 and stirred for one hour at 4° C. The mixture was again centrifuged (15,000 rpm, 30 minutes) and the protein pellet was collected. The protein was resuspended by sonication and the protein was solubilized with urea (4M final concentration). This solution was centrifuged to remove any particulate material (15,000 rpm, 30 minutes) and the supernatant was collected and dialyzed against three, one liter changes of 10 mM Tris-HCl, pH 7.5, 1 mM EDTA to remove the urea. The protein solution was again centrifuged to remove any particulate material (15,000 rpm, 30 minutes) and the supernatant which contained the C13 protein, was collected and used for assays.

Example 4. T Cell Assay

The ability of the C13 protein to induce a cytoxic T cell response in an in vitro assay was compared to that of other proteins also of A/PR/8/34 origin. The other proteins are herein identified as:

| | |
|---|---|
| C7 | (complete HA) |
| Delta 7 | (HA1 and 80 N terminal residues of HA2) |
| C36 | (HA2) |
| Delta 13 | (80 N terminal residues of NS1 and 80 N terminal residues of HA2) |
| NS1 | (NS1) |
| NS2 | (NS2) |
| M30 | (NS1 and M) (See, Example 1) |

Molecules comprising the coding sequence for each of these was derived as described by Young et al., in the Origin of Pandemic Influenza Viruses, 1983, edit. by W.G. Laver, Elsevier Science Publishing Co. and were expressed in pAs1deltaEH substantially as described above. The protein was produced substantially as described above except that following resuspension of the bacterial pellet, lysozyme treatment, sonication and centrifugation, the NS1 protein was contained in the supernatant.

The supernatant was made 100 mM $MgCl_2$ and stirred for one hour at 4° C. The solution was then centrifuged (15,000 rpm, 30 minutes) to pellet the NS1. The pellet was resuspended in buffer A and again treated with 100 mM $MgCl_2$ to reprecipitate the NS1 protein. Following a recentrifugation, the pellet was resuspended in buffer A and dialyzed against three one liter changes of 10 mM Tris-HCl, pH 7.5, 1 mM EDTA. The solution was then centrifuged again to remove any particulate material and the supernatant containing the NS1 protein was collected and used for assays.

The cytoxic T cell assay was carried out substantially as follows. Spleen cells were isolated from virus-immune or non-immune mice and cultured in vitro. Cells were subdivided and exposed to various antigens for 90 mins in vitro. The antigens were then removed by repeated washing of cells and the cells were then cultured for 5 days to allow expansion of stimulated populations. Stimulated cells, that is, effector cells, were mixed with virus-infected or non-infected P815 target cells which had been pre-loaded with $^{51}Cr$. Significant release of $^{51}Cr$ into the culture medium indicated a presence of secondary cytotoxic T cells (2° CTL), which had been generated by the in vitro stimulation with antigen. The specificity of killing was examined in two ways: (1) target cells infected with different antigens were tested for killing; and, 2) spleen cells were isolated from mice which had been immunized with different viruses. The linearity of the assay was also examined using different target: effector cell ratios and using different amounts of antigen, as indicated in the following table, which show illustrative results. Results are listed below indicated effector target cell ratios which were 30:1 and 10:1.

Values in the tables are expressed as the percent of $^{51}Cr$ released into the medium compared to the total amount of $^{51}Cr$ in cells as determined by detergent solubilization of cells. Significant positive results are enclosed in boxes. Viruses used in the assays were:

| | |
|---|---|
| A/PR/8/34 (HIN1) | ("PR8") |
| A/Port Chalmers/174 (H3N2) | ("A/PC") |
| A/Brazil/178 (H2N1) | ("A/B2)") |
| A/Singapore/157 (H2N2). | ("A/Sing"). |

TABLE 1

STIMULATION OF 2° CTL RESPONSE BY E.COLI-DERVIED POLYPEPTIDES

| Antigen used for 2° Stimulation | PR8-P815 | | Uninfected-P815 | |
|---|---|---|---|---|
| | 30 | 10 | 30 | 10 |
| PR8 | 36.0 | 27.2 | 0.2 | -5.2 |
| C13 24 μg/ml | 10.7 | 10.7 | 0.5 | -4.4 |
| 12 μg/ml | 10.6 | 8.1 | 0.5 | -3.6 |
| 6 μg/ml | 9.5 | 4.4 | 1.4 | -4.1 |
| $NS_2$ 24μg/ml | 0.4 | 2.8 | -4.0 | -2.7 |
| 12μg/ml | -1.8 | -1.5 | 0.2 | -4.1 |
| 6μg/ml | -0.5 | -2.0 | -0.3 | -5.2 |
| M30 24μg/ml | -1.0 | -3.9 | -1.6 | -3.3 |
| 12μg/ml | -1.8 | -1.2 | -1.9 | 1.1 |
| 6μg/ml | 5.7 | -1.5 | -3.6 | -6.2 |
| No | -2.7 | -4.1 | -4.7 | -4.0 |

TABLE 2

STIMULATION OF 2° CTL RESPONSE BY E. COLI-DERIVED POLYPEPTIDES*

| Antigen used for 2° Stimulation | PR8-P815 | | Uninfected -P815 | |
|---|---|---|---|---|
| | 30 | 10 | 30 | 10 |
| PR8** | 60.5 | 37.4 | 6.7 | 2.6 |
| $NS_1$ | - 5.2 | - 8.7 | 4.3 | 0.0 |
| C13 | 15.0 | - 1.1 | 0.5 | -0.5 |
| Δ13 | - 8.6 | -10.1 | - 1.3 | 0.0 |
| Δ 7 | - 3.2 | - 7.4 | 2.9 | -0.9 |
| No | - 5.4 | - 2.0 | 1.9 | 3.2 |

* Spleen cells taken from PR8-immune mice were stimulated in vitro with antigens (5μg/ml).
** PR8-infected syngeneic spleen cells.

TABLE 3

VIRUS SPECIFICITY OF CTL STIMULATED BY POLYPEPTIDE C13

| Effector 1°* | 2° | PR8-P815 30 | PR8-P815 10 | A/PC-P815 30 | A/PC-P815 10 | Uninfected-P815 30 | Uninfected-P815 10 |
|---|---|---|---|---|---|---|---|
| PR8 | PR8 | 80.5 | 59.4 | 72.8 | 55.8 | 4.2 | 0.2 |
| | A/PC | 77.9 | 76.1 | 74.2 | 59.3 | 2.3 | 2.1 |
| | C13 24μg/ml | 33.2 | 14.8 | -0.4 | - 0.7 | 1.7 | 1.3 |
| | 12μg/ml | 11.0 | 3.9 | 2.4 | - 2.4 | 4.4 | - 1.4 |
| | 6μg/ml | 8.0 | 1.2 | 2.3 | 2.3 | 0.8 | 0.4 |
| A/PC | PR8 | 96.0 | 72.9 | 74.3 | 57.5 | 9.8 | 1.8 |
| | A/PC | 92.3 | 75.6 | 85.1 | 80.0 | 10.2 | 3.8 |
| | C13 24μg/ml | 6.7 | 1.0 | 4.3 | -5.0 | 2.6 | 0.2 |
| | 12μg/ml | 4.6 | - 1.2 | 1.2 | -3.7 | 2.4 | -0.3 |
| | 6μg/ml | 5.2 | 1.5 | - 0.2 | -6.3 | 3.1 | -1.5 |

* Spleen cells were isolated from mice which had been pre-immunized with the indicated virus

TABLE 4

VIRUS SPECIFICITY OF Tc STIMULATED BY POLYPEPTIDE C13

| Effector 1° | Effector 2° | A/PR/8(H1N1) 30 | A/PR/8(H1N1) 10 | A/BZ 30 | A/BZ 10 | A/SING 30 | A/SING 10 | A/PC 30 | A/PC 10 | Uninfected 30 | Uninfected 10 |
|---|---|---|---|---|---|---|---|---|---|---|---|
| PR8 | PR8 | 76.5 | 77.0 | 82.8 | 75.5 | 30.6 | 17.8 | 92.8 | 79.0 | 8.4 | 3.0 |
| | C13 48µg/ml | 60.9 | 42.6 | 65.5 | 41.3 | 5.8 | 0.0 | 5.7 | 7.5 | 4.1 | 3.0 |
| | 24µg/ml | 70.1 | 46.5 | 63.5 | 46.2 | 9.5 | 5.6 | 9.2 | 9.3 | 8.8 | 0.6 |
| | 12µg/ml | 50.7 | 24.0 | 45.0 | 18.4 | 2.0 | 4.4 | 10.3 | 8.5 | 2.0 | 1.1 |
| No | PR8 | 10.6 | - 0.8 | 14.0 | 16.3 | 15.3 | 6.7 | 13.2 | 5.6 | 2.0 | 1.5 |
| | C13 48µg/ml | -0.8 | - 0.6 | 8.4 | 3.6 | 4.6 | 3.7 | 2.5 | 2.3 | 2.3 | - 2.9 |
| | 24µg/ml | 0.7 | 0.3 | 7.6 | 7.3 | 5.2 | 6.7 | 4.8 | 4.6 | 5.1 | - 2.0 |
| | 12µg/ml | 1.8 | - 0.9 | 10.9 | 2.9 | 3.6 | 2.5 | 4.9 | 2.9 | 5.1 | 8.3 |

As indicated in the preceding tables, C13 induces a secondary cytotoxic T cell response using immune spleen cells from mice previously infected with sublethal doses of PR8 virus. All other peptide derivatives that were studied, including NS1, delta7, delta13, M30, NS2 and C36, failed to induce such a response, as have certain hemagglutinin constructs. The response to the C13 peptide is dose dependent and levels from 5 micrograms per ml through 24 micrograms per ml induced secondary cytotoxic T cell responses.

The viral specificity of the observed responses is striking in that C13 stimulated immune spleen cells from mice previously infected with H1N1 virus but did not stimulate immune spleen cells in mice previously infected with H3N2 virus (A/PC). This is unlike the subtype cross-reactive cytotoxic T lymphocyte responses observed when stimulating the same spleen cells with live virus due, at least in part, to the cross-reactive internal antigens. In addition the stimulation by C13 is cross-reactive among virus strains in the H1N1 subtype; PR8 immune spleen cells stimulated by C13 in vitro were able to recognize and kill target cells infected with PR8 (H1N1 strain from 1934) as well as target cells infected with the A/Brazil (H1N1 strain of 1978) over a dosage range of 12 micrograms through 48 micrograms and at a high degree of cytotoxic activity.

Based on substantial data showing that cytotoxic T lymphocytes appear to contribute to recovery from influenza virus infection in mouse systems and that such lymphocyte responses can be detected in both immune mice and humans (see, Ennis et al., Microbiology - 1984, pages 427-430, Amer. Soc. Microbiology), the ability of the C13 protein to induce such cytoxic T cell response across strains indicates its utility, and the utility of the HA2 immunogenic determinant, to induce an immune response which will resist influenza virus infection; the response is semi-universal in that it is subtype, but not strain, specific.

The invention and its preferred embodiments are fully disclosed above. However, the invention is not limited to such specifically disclosed embodiments. Rather, it encompasses all modifications and variations coming within the scope of the following claims.

## Claims

1. A vaccine for stimulating protection in animals against infection by influenza virus which comprises a polypeptide, other than an HA protein, having an immunogenic determinant of the HA2 subunit of an HA protein genetically fused to a polypeptide other than a polypeptide of E. coli origin, provided that the immunogenic determinant of the HA2 subunit is encoded by an amino acid coding sequence other than the amino acid coding sequence which encodes only the N-terminal 1-80 amino acids of the HA2 subunit.

2. The vaccine of claim 1 in which the vaccine comprises an immunogenic determinant of the HA2 subunit of the HA protein of one or more of the H1, H2 and H3 subtypes of type A influenza virus.

3. The vaccine of claim 1 in which the immunogenic determinant is carried on a fusion protein having the HA2 subunit fused to a polypeptide other than a polypeptide of E. coli origin, which causes the HA2 subunit to assume an immunogenic configuration.

4. The vaccine of claim 3 in which the polypeptide fused to the HA2 subunit comprises N-terminal amino acids of the NS1 protein and in which said N-terminal amino acids are fused to the N-terminal of the HA2 subunit.

5. The vaccine of claim 4 in which the polypeptide fused to the HA2 subunit comprises about 80 N-terminal amino acids of the NS1 protein.

6. The vaccine of claim 5 in which the HA2 subunit is derived from the H1, H2 or H3 subtype of type A influenza virus.

7. The vaccine of claim 5 in which the immunogenic determinant is carried on the C13 protein comprising 81 N-terminal amino acids of the NS1 protein fused to the N-terminal of the HA2 subunit.

8. A polypeptide, other than HA protein, which comprises an immunogenic determinant of the HA2 subunit genetically fused to a polypeptide other than a polypeptide of E. coli origin, provided that the

immunogenic determinant of the HA2 subunit is encoded by an amino acid coding sequence other than the amino acid coding sequence which encodes the N-terminal 1-80 amino acids of the HA2 subunit.

**9.** The polypeptide of claim 8 which is a fusion protein having the HA2 subunit fused to a polypeptide which causes the HA2 subunit to assume an immunogenic configuration.

**10.** The polypeptide of claim 8 in which the polypeptide fused to the HA2 subunit comprises N-terminal amino acids of the NS1 protein and in which said N-terminal amino acids are fused to the N-terminal of the HA2 subunit.

**11.** The polypeptide of claim 10 in which the polypeptide fused to the HA2 subunit comprises about 80 N-terminal amino acids of the NS1 protein.

**12.** The polypeptide of claim 11 in which the HA2 subunit is derived from the H1, H2 or H3 subtype of type A influenza virus.

**13.** C13 protein comprised of 81 N-terminal amino acids of NS1 protein fused to the N-terminal end of the HA2 subunit.

**14.** A DNA molecule comprising a coding sequence for the polypeptide of claim 8.

**15.** A DNA molecule comprising a coding sequence for the polypeptide of claim 9.

**16.** A DNA molecule comprising a coding sequence for the polypeptide of claim 10.

**17.** A DNA molecule comprising a coding sequence for the polypeptide of claim 11.

**18.** A DNA molecule comprising a coding sequence for the polypeptide of claim 12.

**19.** A DNA molecule comprising a coding sequence 30 for the polypeptide of claim 13.

**20.** An expression vector for the protein of claim 13.

**21.** A microorganism or cell transformed with the DNA molecule of claim 14.

**22.** The microorganism of claim 21 which is an E. coli.

**23.** A process for preparing a hybrid polypeptide which can stimulate an immune response in an animal to infection by influenza virus which comprises genetically fusing an immunogenic determinant of the HA2 subunit of the influenza virus HA protein to a polypeptide, other than a polypeptide of E. coli origin, which causes the HA2 immunogenic determinant to assume an immunogenic configuration, provided that the immunogenic determinant of the HA2 subunit is encoded by an amino acid coding sequence other than the amino acid coding sequence which encodes the N-terminal 1-80 amino acids of the HA2 subunit.

**24.** A vaccine for stimulating protection in animals against infection by influenza virus which comprises a polypeptide, other than an HA protein, having an immunogenic determinant of the HA2 subunit of an HA protein, synthetically fused to a polypeptide, other than keyhole limpet hemocyanin.

**25.** A polypeptide, other than an HA protein, which comprises an immunogenic determinant of the HA2 subunit of an HA protein synthetically fused to a polypeptide other than keyhole limpet hemocyanin.

**26.** A process for preparing a hybrid polypeptide which can stimulate an immune response in an animal to infection by influenza virus which comprises fusing by synthetic techniques, an immunogenic determinant of the HA2 subunit of the influenza virus HA protein to a polypeptide, other than keyhole limpet hemocyanin, which causes the HA2 immunogenic determinant to assume an immunogenic configuration.

Claims for the following Contracting State: AT

1. A process for preparing a hybrid polypeptide which can stimulate an immune response in an animal to infection by influenza virus which comprises genetically fusing an immunogenic determinant of the HA2 subunit of the influenza virus HA protein to a polypeptide, other than a polypeptide of E. coli origin, which causes the HA2 immunogenic determinant to assume an immunogenic configuration, provided that the immunogenic determinant of the HA2 subunit is encoded by an amino acid coding sequence other than the amino acid coding sequence which encodes only the N-terminal 1-80 amino acids of the HA2 subunit.

2. The process of claim 1 wherein the hybrid polypeptide which can stimulate the immune response to infection by influenza virus is prepared by expressing, in a microorganism or cell, a coding sequence comprising a region which codes for the HA2 immunogenic determinant and a region which codes for the polypeptide which causes the HA2 immunogenic determinant to assume an immunogenic configuration.

3. The process of claim 2 in which the polypeptide fused to the HA2 subunit comprises N-terminal amino acids of the NS1 protein and in which said N-terminal amino acids are fused to the N-terminal of the HA2 subunit.

4. The process of claim 3 in which the polypeptide fused to the HA2 subunit comprises about 80 N-terminal amino acids of the NS2 protein.

5. The process of claim 4 in which the HA2 subunit is derived from the H1, H2 or H3 subtype of type A influenza virus.

6. The process of claim 5 which comprises fusing the first 81 amino acids of the NS1 protein, through a serine and an arginine of HA1 origin, to the entire HA2 subunit to prepare the C13 protein.

7. A process for preparing a hybrid polypeptide which can stimulate an immune response in an animal to infection by influenza virus which comprises fusing by synthetic techniques, an immunogenic determinant of the HA2 subunit of the influenza virus HA protein to a polypeptide, other than keyhole limpet hemocyanin, which causes the HA2 immunogenic determinant to assume an immunogenic configuration.

**Revendications**

1. Vaccin pour stimuler une protection chez des animaux contre une infection par un virus de l'influenza, caractérisé en ce qu'il comprend un polypeptide autre qu'une protéine HA ayant un déterminant immunogène de la sous-unité HA2 d'une protéine HA fusionnée génétiquement à un polypeptide autre d'un polypeptide d'origine E. coli, à condition que le déterminant immunogène de la sous-unité HA2 soit codé par une séquence codante d'acide aminé autre que la séquence codante d'acide aminé qui ne code que pour les acides aminés 1 à 80 à N-terminal de la sous-unité HA2.

2. Vaccin suivant la revendication 1, caractérisé en ce que le vaccin comprend un déterminant immunogène de la sous-unité HA2 de la protéine HA d'un ou plusieurs des sous-types H1, H2 ou H3 du virus de l'influenza de type A.

3. Vaccin suivant la revendicaiton 1, caractérisé en ce que le déterminant immunogène est porté sur une protéine de fusion ayant la sous-unité HA2 fusionnée à un polypeptide autre qu'un polypeptide d'origine E. coli, qui provoque la prise d'une configuration immunogène par la sous-unité HA2.

4. Vaccin suivant la revendication 3, caractérisé en ce que le polypeptide fusionné à la sous-unité HA2 comprend des acides aminés à N-terminal de la protéine NS1 et que ces acides aminés à N-terminal sont fusionnés au N-terminal de la sous-unité HA2.

5. Vaccin suivant la revendication 4, caractérisé en ce que le polypeptide fusionné à la sous-unité HA2

comprend environ 80 acides aminés à N-terminal de la protéine NS1.

6. Vaccin suivant la revendication 5, caractérisé en ce que la sous-unité HA2 est dérivée du sous-type H1, H2 ou H3 du virus de l'influenza de type A.

7. Vaccin suivant la revendication 5, caractérisé en ce que le déterminant immunogène est porté sur la protéine C13 comprenant 81 acides aminés à N-terminal de la protéine NS1 fusionnée au N-terminal de la sous-unité HA2.

8. Polypeptide autre que la protéine HA caractérisé en ce qu'il comprend un déterminant immunogène de la sous-unité HA2 génétiquement fusionnée à un polypeptide autre qu'un polypeptide d'origine E. coli, à condition que le déterminant immunogène de la sous-unité HA2 soit codé par une séquence codante d'acides aminés autre que la séquence codante d'acides aminés qui code pour les acides aminés 1 à 80 à N-terminal de la sous-unité HA2.

9. Polypeptide suivant la revendication 8, caractérisé en ce qu'il s'agit d'une protéine de fusion ayant la sous-unité HA2 fusionnée à un polypeptide qui provoque la prise d'une configuration immunogène par la sous-unité HA2.

10. Polypeptide suivant la revendication 8, caractérisé en ce que le polypeptide fusionné à la sous-unité HA2 comprend des acides aminés à N-terminal de la protéine NS1 et que les acides aminés à N-terminal sont fusionnés au N-terminal de la sous-unité HA2.

11. Polypeptide suivant la revendication 10, caractérisé en ce que le polypeptide fusionné à la sous-unité HA2 comprend environ 80 acides aminés à N-terminal de la protéine NS1.

12. Polypeptide suivant la revendication 11, caractérisé en ce que la sous-unité HA2 dérive du sous-titre H1, H2 ou H3 du virus de l'influenza de type A.

13. Protéine C13 caractérisée en ce qu'elle est composée de 81 acides aminés à N-terminal de la protéine NS1 fusionnée à l'extrémité N-terminal de la sous-unité HA2.

14. Molécule d'ADN caractérisée en ce qu'elle comprend une séquence codante pour le polypeptide de la revendication 8.

15. Molécule d'ADN caractérisée en ce qu'elle comprend une séquence codante pour le polypeptide de la revendication 9.

16. Molécule d'ADN caractérisée en ce qu'elle comprend une séquence codante pour le polypeptide de la revendication 10.

17. Molécule d'ADN caractérisée en ce qu'elle comprend une séquence codante pour le polypeptide de la revendication 11.

18. Molécule d'ADN caractérisée en ce qu'elle comprend une séquence codante pour le polypeptide de la revendication 12.

19. Molécule d'ADN caractérisée en ce qu'elle comprend une séquence codante 30 pour le polypeptide de la revendication 13.

20. Vecteur d'expression caractérisé en ce qu'il s'agit d'un vecteur pour la protéine de la revendication 13.

21. Microorganisme ou cellule transformé caractérisé en ce qu'il s'est transformé avec la molécule d'ADN suivant la revendication 14.

22. Microorganisme suivant la revendication 21, caractérisé en ce qu'il s'agit de E. coli.

23. Procédé pour préparer un polypeptide hybride qui peut stimuler une réponse immune chez un animal à

une infection par un virus de l'influenza, caractérisé en ce qu'il comprend la fusion génétique d'un déterminant immunogène de la sous-unité HA2 de la protéine HA du virus de l'influenza à un polypeptide, autre qu'un polypeptide d'origine E. coli, qui provoque la prise de la configuration immunogène par le déterminant immunogène de HA2, à condition que le déterminant immunogène de la sous-unité HA2 soit codé par une séquence codante d'acides aminés autre que la séquence codante d'acides aminés qui code pour les acides aminés 1 à 80 à N-terminal de la sous-unité HA2.

**24.** Vaccin pour stimuler une protection chez les animaux contre une infection par un virus de l'influenza, caractérisé en ce qu'il comprend un polypeptide, autre qu'une protéine HA, ayant un déterminant immunogène de la sous-unité HA2 d'une protéine HA, fusionnée synthétiquement à un polypeptide, autre que l'hémocyanine de patelle en trou de serrure.

**25.** Polypeptide, autre qu'une protéine HA, caractérisé en ce qu'il comprend un déterminant immunogène de la sous-unité HA2 d'une protéine HA fusionnée synthétiquement à un polypeptide autre que l'hémocyanine de patelle en trou de serrure.

**26.** Procédé pour préparer un polypeptide hybride qui peut stimuler une réponse immune chez un animal à une infection par un virus de l'influenza, caractérisé en ce qu'il comprend la fusion par des techniques synthétique, d'un déterminant immunogène de la sous-unité HA2 de la protéine HA du virus de l'influenza à un polypeptide, autre que l'hémocyanine de patelle en trou de serrure, qui provoque la prise d'une configuration immunogène par le déterminant immunogène de HA2.

Revendications pour l'Etat contractant suivant: AT

**1.** Procédé pour préparer un polypeptide hybride qui peut stimuler une réponse immune chez un animal à une infection par un virus de l'influenza, caractérisé en ce qu'il comprend la fusion génétique d'un déterminant immunogène de la sous-unité HA2 de la protéine HA du virus de l'influenza à un polypeptide, autre d'un polypeptide d'origine E. coli, qui provoque la prise de la configuration immunogene par le déterminant immunogène de HA2, à condition qu ele déterminant immunogène de la sous-unité HA2 soit codé par une séquence codante d'acides aminés autre que la séquence codante d'acides aminés qui code seulement pour les acides aminés 1 à 80 à N-terminal de la sous-unité HA2.

**2.** Procédé suivant la revendication 1, caractérisé en ce que le polypeptide hybride qui peut stimuler une réponse immune à une infection par un virus de l'influenza est préparé par expression dans un microorganisme ou une cellule d'une séquence codante comprenant une région qui code pour le déterminant immunogène HA2 et une région qui code pour le polypeptide qui provoque la prise de configuration immunogène par le déterminant immunogene de HA2.

**3.** Procédé suivant la revendication 2, caractérisé en ce que le polypeptide fusionné à la sous-unité HA2 comprend les acides aminés N-terminaux de la protéine NS1 et dans lequel les acides aminés N-terminaux sont fusionnés aux acides aminés N-terminaux de la sous-unité HA2.

**4.** Procédé suivant la revendication 3, caractérisé en ce que le polypeptide fusionné à la sous-unité HA2 comprend environ 80 acides aminés N-terminaux de la protéine NS1.

**5.** Procédé suivant la revendication 4, caractérisé en ce que la sous-unité HA2 dérive du sous-type H1, H2 ou H3 du virus de l'influenza de type A.

**6.** Procédé suivant la revendication 5, caractérisé en ce qu'il comprend la fusion des 81 premiers acides aminés de la protéine NS1, par l'intermédiaire d'une unité sérine et d'une unité arginine d'origine HA1 à la sous-unité HA2 entière pour préparer la protéine C13.

**7.** Procédé pour préparer un polypeptide hybride qui peut stimuler une réponse immune chez un animal à une infection par un virus de l'influenza, caractérisé en ce qu'il comprend la fusion par des techniques synthétiques, d'un déterminant immunogène de la sous-unité HA2 de la protéine HA du virus de l'influenza à un polypeptide, autre que l'hémocyanine de patelle en trou de serrure, qui provoque la prise d'une configuration immunogène par le déterminant immunogène de HA2.

## Ansprüche

1. Impfstoff zur Stimulierung des Schutzes gegen eine Infektion durch Influenzavirus in Tieren, der ein anderes Polypeptid als ein HA-Protein umfaßt, das eine immunogene Determinante der HA2-Untereinheit von einem HA-Protein aufwe ist, die genetisch mit einem anderen als einem von E. coli stammenden Polypeptid fusioniertist, mit der Maßgabe, daß die immunogene Determinante der HA2-Untereinheit von einer anderen, eine Aminosäure codierenden Sequenz codiert wird, als der eine Aminosäure codierenden Sequenz, die nur die N-terminalen 1-80 Aminosäuren der HA2-Untereinheit codiert.

2. Impfstoff nach Anspruch 1, in dem der Impfstoff eine immunogene Determinante der HA2-Untereinheit des HA-Proteins von einer oder mehreren der H1-, H2- und H3-Unterarten des Influenzavirus Typ A umfaßt.

3. Impfstoff nach Anspruch 1, in dem die immunogene Determinante in einem Fusionsprotein vorliegt, in dem die HA2-Untereinheit an ein anderes als ein von E. coli stammendes Polypeptid fusioniert ist, was verursacht, daß die HA2-Untereinheit eine immunogene Konfiguration annimmt.

4. Impfstoff nach Anspruch 3, in dem das an die HA2-Untereinheit fusionierte Polypeptid N-terminale Aminosäuren des NS1-Proteins umfaßt und die N-terminalen Aminosäuren mit dem N-terminalen Ende der HA2-Untereinheit fusioniert sind.

5. Impfstoff nach Anspruch 4, in dem das mit der HA2-Untereinheit fusionierte Polypeptid etwa 80 N-terminale Aminosäure des NS1-Proteins umfaßt.

6. Impfstoff nach Anspruch 5, in dem die HA2-Untereinheit von der H1-, H2- oder H3-Unterart des Influenzavirus Typ A stammt.

7. Impfstoff nach Anspruch 5, in dem die immunogene Determinante in dem C13-Protein vorliegt, das 81 N-terminale Aminosäuren des NS1-Proteins, fusioniert mit dem N-terminalen Ende der HA2-Untereinheit, umfaßt.

8. Polypeptid, ausgenommen HA-Protein, das eine immunogene Determinante der HA2-Untereinheit, genetisch mit einem anderen als einem von E. coli stammenden Polypeptid fusioniert, umfaßt, mit der Maßgabe, daß die immunogene Determinante der HA2-Untereinheit von einer anderen eine Aminosäure codierenden Sequenz codiert wird, als der eine Aminosäure codierenden Sequenz, die die N-terminalen 1-80 Aminosäuren der HA2-Untereinheit codiert.

9. Polypeptid nach Anspruch 8, das ein Fusionsprotein ist, das die mit einem Polypeptid fusionierte HA2-Untereinheit aufweist, was verursacht, daß die HA2-Untereinheit eine immunogene Konfiguration annimmt.

10. Polypeptid nach Anspruch 8, in dem das an die HA2-Untereinheit fusionierte Polypeptid N-terminale Aminosäuren des NS1-Proteins umfaßt und die N-terminalen Aminosäuren mit dem N-terminalen Ende der HA2-Untereinheit fusioniert sind.

11. Polypeptid nach Anspruch 10, in dem das mit der HA2-Untereinheit fusionierte Polypeptid etwa 80 N-terminale Aminosäuren des NSI-Proteins umfaßt.

12. Polypeptid nach Anspruch 11, in dem die HA2-Untereinheit von der H1-, H2- oder H3-Untereinheit des Influenzavirus Typ A abstammt.

13. C13-Protein, das 81 N-terminalen Aminosäuren des NS1-Proteins, fusioniert mit dem N-terminalen Ende der HA2-Untereinheit, umfaßt.

14. DNA-Molekül, das eine codierende Sequenz für das Polypeptid nach Anspruch 8 umfaßt.

15. DNA-Molekül, das eine codierende Sequenz für das Polypeptid nach Anspruch 9 umfaßt.

16. DNA-Molekül, das eine codierende Sequenz für das Polypeptid nach Anspruch 10 umfaßt.

17. DNA-Molekül, das eine codierende Sequenz für das Polypeptid nach Anspruch 11 umfaßt.

18. DNA-Molekül, das eine codierende Sequenz für das Polypeptid nach Anspruch 12 umfaßt.

19. DNA-Molekül, das eine codierende Sequenz für das Polypeptid nach Anspruch 13 umfaßt.

20. Expressionsvektor für das Protein nach Anspruch 13.

21. Mikroorganismus oder Zelle, transformiert mit dem DNA-Molekül von Anspruch 14.

22. Mikroorganismus nach Anspruch 21, der E. coli ist.

23. Verfahren zur Herstellung eines Hybridpolypeptids, das in einem Tier eine Immunantwort auf eine Infektion durch Influenzavirus stimulieren kann, umfassend die genetische Fusionierung einer immunogenen Determinante der HA2-Untereinheit des Influenzavirus-HA-Proteins an ein anderes als ein von E. coli stammendes Polypeptid, was verursacht, daß die HA2-immunogene Determinante eine immunogene Konfiguration annimmt, mit der Maßgabe, daß die immunogene Determinante der HA2-Untereinheit von einer anderen, eine Aminosäure codierenden Sequenz codiert wird, als der eine Aminosäure codierenden Sequenz, die die N-terminalen 1-80 Aminosäuren der HA2-Untereinheit codiert.

24. Impfstoff zur Stimulierung des Schutzes gegen eine Infektion von Influenzavirus in Tieren, das ein anderes Polypeptid als ein HA-Protein umfaßt, das eine immunogene Determinante der HA2-Untereinheit von einem HA-Protein aufweist, synthetisch an ein anderes Polypeptid als "keyhole limpet" Hämocyanin fusioniert.

25. Polypeptid, ausgenommen ein HA-Protein, das eine immunogene Determinante der HA2-Untereinheit eines HA-Proteins, synthetisch an ein anderes Polypeptid als "keyhole limpet" Hämocyanin fusioniert, umfaßt.

26. Verfahren zur Herstellung eines Hybridpolypeptids, das in einem Tier eine Immunantwort auf eine Infektion durch Influenzavirus stimulieren kann, umfassend die Fusionierung durch synthetische Techniken einer immunogenen Determinante der HA2-Untereinheit des Influenzavirus-HA-Proteins an ein anderes Polypeptid, als "keyhole limpet" Hämocyanin, was verursacht, daß die HA2 immunogene Determinante eine immunogene Konfiguration annimmt.

Patentansprüche für folgende Vertragsstaat: AT

1. Verfahren zur Herstellung eines Hybridpolypeptids, das in einem Tier eine Immunantwort auf eine Infektion durch Influenzavirus stimulieren kann, umfassend die genetische Fusionierung einer immunogenen Determinante der HA2-Untereinheit des Influenzavirus-HA-Proteins an ein anderes als ein von E. coli stammendes Polypeptid, was verursacht, daß die HA2-immunogene Determinante eine immunogene Konfiguration annimmt, mit der Maßgabe, daß die immunogene Determinante der HA2-Untereinheit von einer anderen, eine Aminosäure codierenden Sequenz codiert wird, als der eine Aminosäure codierenden Sequenz, die die N-terminalen 1-80 Aminosäuren der HA2-Untereinheit codiert.

2. Verfahren nach Anspruch 1, in dem das Hybridpolypeptid, das eine Immunantwort auf eine Infektion durch Influenzavirus stimulieren kann, in einem Mikroorganismus oder einer Zelle, durch Expression, einer codierenden Sequenz, umfassend einen Bereich, der für die HA2-immunogene Determinante codiert, und einen Bereich, der für ein Polypeptid codiert, hergestellt wird, was verursacht, daß die HA2-immunogene Determinante eine immunogene Konfiguration annimmt.

3. Verfahren nach Anspruch 2, in dem das mit der HA2-Untereinheit fusionierte Polypeptid N-terminale Aminosäuren des NS1-Proteins umfaßt, und in dem die N-terminalen Aminosäuren mit dem N-terminalen Ende der HA2-Untereinheit fusioniert sind.

4. Verfahren nach Anspruch 3, in dem das mit der HA2-Untereinheit fusionierte Polypeptid etwa 80 N-terminale Aminosäuren des NS2-Proteins umfaßt.

5. Verfahren nach Anspruch 4, in dem die HA2-Untereinheit von der H1-, H2- oder H3-Untereinheit des Influenzavirus Typ A stammt.

6. Verfahren nach Anspruch 5, das die Fusionierung der ersten 81 Aminosäuren des NS1-Proteins, durch ein Serin und ein Arginin der HA1-Untereinheit, mit der vollständigen HA2-Untereinheit zur Herstellung des C13-Proteins umfaßt.

7. Verfahren zur Herstellung eines Hybridpolypeptids, das in einem Tier eine Immunantwort auf eine Infektion durch Influenzavirus stimulieren kann, umfassend die Fusionierung durch synthetische Techniken einer immunogenen Determinante der HA2-Untereinheit des Influenzavirus-HA-Proteins an ein anderes Polypeptid als "keyhole limpet" Hämocyanin, was verursacht, daß die HA2 immunogene Determinante eine immunogene Konfiguration annimmt.